# EUROPÄISCHE PATENTSCHRIFT

(11) **EP 2 803 655 B1**
(45) Veröffentlichungstag und Bekanntmachung des Hinweises auf die Patenterteilung: **28.06.2017**
(21) Anmeldenummer: 14162901.4
(22) Anmeldetag: 31.03.2014
(51) Int. Cl.: C07C 1/12, C07C 9/04, C07C 29/151, C07C 31/04, C10G 2/00, C10L 3/06, C12M 1/00, C12P 7/08, C25B 1/04

(54) **System und Verfahren zur Erzeugung von aliphatischen Alkoholen**
System and method for producing aliphatic alcohols
Système et procédé de production d'alcools aliphatiques

(30) Priorität: 16.05.2013 EP 13168043
(43) Veröffentlichungstag der Anmeldung: 19.11.2014
(73) Patentinhaber: Schweitzer, Christian, 04357 Leipzig (DE); Hensel, Robert, 04416 Markkleeberg (DE)
(72) Erfinder: Schweitzer, Christian, 04357 Leipzig (DE); Hensel, Robert, 04416 Markkleeberg (DE)
(74) Vertreter: Kailuweit & Uhlemann Patentanwälte Partnerschaft mbB

(56) Entgegenhaltungen:
- EP-A1- 2 803 654
- WO-A1-2013/029701
- US-A- 5 342 702

## Beschreibung

Die Erfindung betrifft eine Vorrichtung und ein Verfahren zur Erzeugung von aliphatischen Alkoholen, insbesondere Bioethanol.

Bioethanol wird aus nachwachsenden Rohstoffen hergestellt und kommt im Kraftstoffsektor zum Einsatz. Bioethanol kann aus verschiedenen Pflanzen und pflanzlichen Teilen hergestellt werden: aus zuckerhaltigen Pflanzen wie Zuckerrüben und Zuckerrohr, aus stärkehaltigen Pflanzen wie Getreide, Kartoffeln und Mais, sowie aus zellulosehaltigen Rohstoffen wie z. B. Holz.

Zur Gewinnung von Bioethanol werden die in den Pflanzen enthaltenen Kohlenhydrate mit Hilfe von Enzymen und/oder Hefepilzen zu Alkohol vergoren. Für die Produktion von Bioethanol aus zucker- und stärkehaltige Pflanzen gibt es seit geraumer Zeit gängige Verfahren.

Damit Bioethanol-Kraftstoff einen sinnvollen Beitrag zur Energiewirtschaft leisten kann, muss die Herstellung eine positive Treibhausgasbilanz aufweisen. Den hauptsächlichen Anteil der aufgewendeten Primärenergie (Input) macht der Stärkeaufschluss bzw. die Destillation und der nachwachsende und CO₂-neutrale Rohstoff Biomasse aus.

Bei der Fermentation der Rohstoffe und der Verbrennung des Bioethanols wird zwar das Treibhausgas Kohlenstoffdioxid freigesetzt; da jedoch beim Wachstum der Rohstoffpflanzen zuvor die gleiche Menge Kohlenstoffdioxid aus der Atmosphäre durch die Photosynthese gebunden wurde, sind diese chemischen Vorgänge (Photosynthese, Fermentation, Verbrennung) in der Addition CO₂-neutral. Da bei der Produktion der Rohstoffe und bei der Ethanolherstellung zusätzliche Energie benötigt wird, ist der Herstellungsprozess insgesamt nicht CO₂-neutral oder gar klimaneutral.

Aus den zur Ethanolgewinnung nicht benötigten Pflanzenbestandteilen wie Eiweiß, Pflanzenfasern und Fetten entstehen Nahrungs-, Futter- und Düngemittel. Getreideschlempe ist nährstoffreich und wird getrocknet als Futtermittel mit hohem Proteingehalt vermarktet (Trockenschlempe, auch DDGS = "dried distillers grains and solubles"). Zudem kann Dickschlempe als Schweinenassfutter (PDGS) verwendet werden. Bei der Herstellung von einem Liter Bioethanol aus Getreide entsteht so zusätzlich ein Kilogramm Proteinfutter. Vinasse, die bei der Zuckervergärung zurückbleibt, wird agrartechnisch zum Beispiel ebenfalls als Tierfutterzusatz, Biogas-Co-Substrat oder als Düngemittel genutzt.

Eine weitere Möglichkeit für die Verwendung der Schlempe ist die Energiegewinnung durch thermische Verwertung, d. h. die Verbrennung zwecks Dampferzeugung für die Ethanolanlage. Neben einer Senkung der Produktionskosten wird dadurch die Treibhausgasbilanz der Produktion verbessert. Energetisch interessant ist außerdem die Vergärung von Schlempe und anderen Reststoffen der Bioethanolproduktion in Biogasanlagen. Das gewonnene Biogas kann als Energieträger für die Prozesswärme in der Anlage oder wird ins Netz eingespeist. Es kann wie Erdgas als Energieträger in Haushalten oder auch als Kraftstoff genutzt werden.

Aus der DE 10 2007 015 623 A1 ist ein Verfahren zur energetischen Nutzung von landwirtschaftlich erzeugten Rohstoffen vorbekannt. Dabei wird der in einem Heizkraftwerk zum Betreiben einer Dampfturbine gebildete Prozessdampf für verfahrenstechnische nachgeordnete Anwendungen, wie etwa für eine Bioethanolanlage zur Erzeugung von Bioethanol verwendet, wobei für deren energetische Prozesse ein Teil der im Restdampf enthaltenen Energie nutzbar gemacht wird. Weiterhin kann der DE 10 2007 015 623 A1 entnommen werden, dass die bei der Bioethanolerzeugung anfallende Schlempe bei einer Biogaserzeugung verwendet werden kann. Diese Schlempe weist einen hohen Anteil organischer Stoffe auf. Daher ist selbige sehr gut für die Biogaserzeugung geeignet.

Weiterhin offenbart die DE 10 2007 001 614 A1 ein energieautarke Verfahren zur Herstellung von Bioethanol. Dabei wird die in einer Phasentrennung abgetrennte Dicksauermaische zum Zweck der Gewinnung von Biomethan für die Erzeugung von Prozessenergie in einem Blockheizkraftwerk einer parallel betriebenen Methanisierungsstufe zugeführt. Bei einer weiteren Variante des dortigen Verfahrens wird als Prozesswasser das Kondensat aus destilliertem Schlempewasser eingesetzt. Darüber hinaus soll sich das als Schlempewasser anfallende Sumpfprodukt auch wenigstens teilweise zur Gewinnung von Prozesswasser verwenden lassen, indem zur Gewinnung von salzarmem Prozesswasser und salzreichem Konzentrat aus dem Schlempewasser eine Vakuum-Umlaufverdampfungsanlage, eine Mikrofiltrations-, Ultrafiltrations-, Nanofiltrations- sowie Umkehrosmose- und/oder Membrantechniken eingesetzt werden.

Die DE 10 2010 005 818 A1 offenbart ein Verfahren bei dem eine direkte Rückführung des Ablaufs der Biogasanlage zum Zweck des Einmaischens in den Fermenter einer Bioethanolgewinnungsanlage derart vorgenommen wird, dass der Frischwassereinsatz bezogen auf den Gesamtprozess einer wesentlichen Reduzierung unterliegt. Dabei wird aus dem Ablauf der Biogasanlage ein Teilstrom ausgeschleust, mittels eines Membranverfahrens gereinigt und das erhaltene Permeat ganz oder teilweise zum Anmaischen in die Bioethanolgewinnungsanlage eingebracht.

Die DE 10 2008 058 501 B4 offenbart ein Verfahren zum Betreiben einer Anlage zur Herstellung von Bioethanol, bei dem die organische Abfallprodukte des Herstellungsprozesses, verbrannt werden und die so gewonnene Nutzwärme der Anlage selbst wieder zugeführt wird. Die so gewonnene Nutzwärme wird in der Bioethanolgewinnungsanlage selbst wieder verbraucht, wobei sich viele Möglichkeiten finden, Wärme zu nutzen, sei dies nun, um Dampf herzustellen, oder sei dies, um direkt Anlagenteile oder Materialien zu erwärmen.

Die US 5 342 702 A offenbart ein System zur Optimierung von Prozessströmen mit einem Fermenter zur Herstellung von Ethanol sowie einer Methanolsynthesevorrichtung, wobei das bei der Fermentation erzeugte Kohlenstoffdioxid als Edukt der Methanolsynthese dient. Weiterhin ist eine Elektrolysevorrichtung offenbart, welche aus Wasser Wasserstoff und Sauerstoff generiert. Der Wasserstoff wird dabei der Methanolsynthesevorrichtung zugeführt. Des Weiteren wird offenbart, dass durch die Verbrennung von Kohlenwasserstoffen in einem Dampferzeuger Dampf erzeugt wird, welcher mittels Dampfturbine zur Herstellung von Elektrizität genutzt wird. Daneben wird der entstandene Dampf für Erwärmungsprozesse genutzt.

Schließlich offenbart die WO 2010/002469 A1 offenbart ein System mit einer Vorrichtung zur Ethanolsynthese und einer Vorrichtung zur Methansynthese, wobei das bei der Ethanolsynthese erzeugt Kohlenstoffdioxid der Vorrichtung zur Methansynthese zugeführt wird. Der zudem benötigte Wasserstoff wird durch einen Plasmaschmelzofen erzeugt, wobei das bei der Methansynthese anfallende Wasser als Edukt dient. Das Methan selbst wird zur Energieerzeugung verbrannt, wobei das entstehende Kohlenstoffdioxid als Edukt für die Methansynthese dient.

Trotz der aktuellen Fortschritte bei der Optimierung des Energiebedarfs zur Herstellung von Bioethanol, weisen die im bisherigen Stand der Technik bekannten Verfahren noch Potential zu einer verbesserten energieeffizienteren Prozessführung auf.

Die Aufgabe der vorliegenden Erfindung besteht daher darin, eine Vorrichtung und ein Verfahren anzugeben, welche die Nachteile des Stands der Technik überwinden.

Die Aufgabe wird durch eine Vorrichtung gemäß Anspruch 1 und ein Verfahren gemäß Anspruch 7 gelöst. Vorteilhafte Ausgestaltungen sind in den abhängigen Ansprüchen angegeben.

Erfindungsgemäß wird ein System zur Erzeugung von aliphatischen Alkoholen vorgeschlagen, umfassend:
- zumindest eine erste Vorrichtung zur Herstellung von Ethanol, wobei Kohlenstoffdioxid gebildet wird und
- zumindest eine zweite Vorrichtung zur Umsetzung des Kohlenstoffdioxids mittels chemischer Synthese mittels Fischer-Tropsch-Synthese, welche eine exotherme Aufbaureaktion von CO/H2-Gemischen an heterogenen Katalysatoren zu Paraffinen, Alkenen und Alkoholen ist,
   wobei die erste Vorrichtung zur biologischen Herstellung eines ersten aliphatischen Alkohols und die zweite zur Herstellung katalytischen Umsetzung des Kohlendioxids ausgebildet ist, wobei die zweite Vorrichtung zur katalytischen Umsetzung des Kohlendioxids der ersten Vorrichtung zur biologischen Herstellung des Ethanols nachgeschaltet ist sowie eine erste Verbindung von der ersten Vorrichtung zur zweiten Vorrichtung, die es ermöglicht, ein in der ersten Vorrichtung gebildetes Kohlendioxid in die zweite Vorrichtung einzuleiten, wobei
- das System eine zweite Verbindung von der zweiten Vorrichtung zur ersten Vorrichtung aufweist, die es ermöglicht, in der zweiten Vorrichtung gebildetes Wasser in die erste Vorrichtung einzuleiten,
- zumindest eine Vorrichtung zur Dampferzeugung und eine dritte Verbindung, wobei die dritte Verbindung zwischen der Vorrichtung zur Dampferzeugung und der ersten Vorrichtung angeordnet ist und es ermöglicht, den in der Vorrichtung zur Dampferzeugung gebildeten Dampf in die erste Vorrichtung einzuleiten,
- zumindest eine dritte Vorrichtung zur Generation von Wasserstoff , welche eine fünfte Verbindung zur zweiten Vorrichtung aufweist, die es ermöglicht, den Wasserstoff in die zweite Vorrichtung einzubringen, wobei die dritte Vorrichtung eingerichtet ist, aus Wasser Wasserstoff und Sauerstoff zu generieren, wobei die Vorrichtung eingerichtet ist, komprimierten Wasserstoff und Sauerstoff mit einem Druck > 15 bar, welcher in der zweiten Vorrichtung erforderlich ist, zu generieren, wobei
das System eine vierte Verbindung von der ersten Vorrichtung zur Vorrichtung zur Dampferzeugung aufweist, die es ermöglicht, ein in der ersten Vorrichtung gebildetes Kondensat in die Vorrichtung zur Dampferzeugung einzuleiten und wobei die dritte Vorrichtung der zweiten Vorrichtung komprimierten Wasserstoff mit einem Druck > 15 bar zur Verfügung stellt, wobei das System eine vierte Vorrichtung umfasst, welche derart mit der Herstellung des Ethanols verbunden ist, dass der generierte Dampf zum Eintrag thermischer Energie für die Herstellung des Ethanols in der ersten Vorrichtung verwendet wird.

Erfindungsgemäß ist die erste Vorrichtung zur biologischen Herstellung von Ethanol ausgebildet.

Weiter bevorzugt ist die erste Vorrichtung zur biologischen Herstellung von Ethanol durch Gärung ausgebildet. Im biochemischen Prozess zur Ethanolherstellung unter anoxischen Bedingungen wird aus Kohlenhydraten (hauptsächlich Glucose) Ethanol und Kohlendioxid erzeugt. Im Prozess wird die Fähigkeit von Mikroorganismen, bevorzugt Hefen, zur Energiegewinnung durch alkoholische Gärung genutzt, wenn die Umgebungsbedingungen für eine normale Zellatmung fehlen. Bevorzugt handelt es sich bei den Kohlenhydraten um Kohlenhydrate mit 5 oder 6 C-Atomen.

Weiter bevorzugt ist die erste Vorrichtung zur druckarmen (<0,5 bar) Herstellung des Ethanols ausgebildet. Dabei wird beispielsweise mittels alkoholischer Gärung Ethanol erzeugt, wobei das freigesetzte Kohlenstoffdioxid einen Druck <0,5 bar aufweist.

Weiter bevorzugt wird der in der ersten Vorrichtung erzeugte Ethanol durch eine Vorrichtung zur destillativen Abscheidung aus der ersten Vorrichtung entfernt.

Weiter bevorzugt weist die erste Vorrichtung zur Herstellung von Ethanol eine Temperierungseinrichtung auf, welche derart ausgebildet ist, die erste Vorrichtung zu temperieren. Dadurch wird die für den biologischen Prozess zur Herstellung des Ethanols benötigte Reaktionstemperatur eingestellt. In einer Ausgestaltung ist die Temperierungseinrichtung als Wasserbad ausgebildet. Vorteilhaft kann dabei die im System erzeugte thermische Energie an die Temperierungseinrichtung übertragen werden, um eine gleichbeliebend Temperierung zu gewährleisten.

Weiter bevorzugt weist die erste Vorrichtung eine Vorrichtung zur Abtrennung von Ethanol auf. Das Ethanol wird dabei aus dem entstandenen alkoholhaltigen Gemisch mittels eines thermischen Trennverfahrens separiert und anschließend absolutiert. Dabei entstehen Prozessabwässer.

Erfindungsgemäß ist die zweite Vorrichtung eingerichtet, um eine Fischer-Tropsch-Synthese durchzuführen. Die Fischer-Tropsch-Synthese ist eine exotherme Aufbaureaktion von CO/H₂-Gemischen an heterogenen-Katalysatoren zu Paraffinen, Alkenen und Alkoholen. Die Reaktion findet nur unter hohem Druck (20 - 40 bar) und bei einer Temperatur von 200°C - 350°C statt und verläuft nach folgenden allgemeinen Formeln:

nCO + (2n+1)H2 → CnH₂n+2+nH₂O (Alkane)

nCO + (2n)H₂ → CnH₂n+nH₂O (Alkene)

nCO+(2n)H₂ → CnH₂n+1OH+(n-1)H₂O (Alkohole)

Das Verfahren ist für die großtechnische Produktion von Benzin und Ölen von Bedeutung. Das typische Fischer-Tropsch -Produkt nach der Schulz-Flory-Verteilung enthält ca. 10-15% Flüssiggas, 50% Ottokraftstoff, 28% Dieselöle, 6% Weichparaffine und 2% Hartparaffine.

Erfindungsgemäß weist das erfindungsgemäße System eine erste Verbindung von der ersten Vorrichtung zur zweiten Vorrichtung auf, die es ermöglicht, ein in der ersten Vorrichtung gebildetes Kohlendioxid in die zweite Vorrichtung einzuleiten. Erfindungsgemäß weist das erfindungsgemäße System eine zweite Verbindung von der zweiten Vorrichtung zur ersten Vorrichtung auf, die es ermöglicht, in der zweiten Vorrichtung gebildetes Wasser in die erste Vorrichtung einzuleiten. Das in der zweiten Vorrichtung gebildete Wasser kann dabei etwa zum Anmaischen verwendet werden.

Bevorzugt ist die zweite Verbindung von der zweiten Vorrichtung zur ersten Vorrichtung eingerichtet, den in der zweiten Vorrichtung gebildeten Wasserdampf unter Freisetzung von mechanischer Energie zu entspannen und mit reduziertem Druckniveau in die erste Vorrichtung einzuleiten.

Der eingesetzte Dampf ergänzt so die Wasserbilanz im Prozess und gibt zudem Wärme an das Substrat ab, was eine Desinfektion des Substrats bewirkt. Zudem kann durch die Nutzung der thermischen Energie des erzeugten Dampfes teilweise die sonst erforderliche Dampferzeugung durch Verdampfung von Wasser minimiert werden. Der Dampf wird dabei dem Dampfsystem zugeführt und substituiert teilweise die Dampferzeugung. Zudem kann der Dampf zur Generierung von Wasser abgekühlt werden, wodurch eine Zuführung von systemexternem Wasser vermindert werden kann. Gleichfalls kann der Dampf in der Elektrolyse verwendet werden, wodurch ein neuer Ansatz zur Optimierung der Elektrolysekapazität gewährleistet ist.

Erfindungsgemäß umfasst das erfindungsgemäße System zumindest eine Vorrichtung zur Dampferzeugung und eine dritte Verbindung, wobei die dritte Verbindung zwischen der Vorrichtung zur Dampferzeugung und der ersten Vorrichtung angeordnet ist und es ermöglicht, den in der Vorrichtung zur Dampferzeugung gebildeten Dampf in die erste Vorrichtung einzuleiten. Dabei wird der Vorrichtung zur Dampferzeugung beispielsweise chemische gebundene Energie in Form von Kohlenwasserstoffen zugeführt und mit Sauerstoff, beispielsweise aus vorbehandelter Umgebungsluft verbrannt. Beispielhaft soll an dieser Stelle die Oxidation von im Brennstoff enthaltenem reinen Kohlenstoff mit Sauerstoff stehen. Nach der Verbrennung entsteht ein stickoxidhaltiges Abgasgemisch. Die freie thermische Energie kann an vollentsalztes Wasser übertragen werden, wobei Wasserdampf entsteht, der als Übertragungsmedium für thermische und mechanische Energie in weitere Prozesse dienen kann.

Erfindungsgemäß weist das erfindungsgemäße System eine vierte Verbindung von der ersten Vorrichtung zur Vorrichtung zur Dampferzeugung auf, die es ermöglicht, ein in der ersten Vorrichtung gebildetes Kondensat in die Vorrichtung zur Dampferzeugung einzuleiten. Dabei wird in der ersten Vorrichtung die Energie aus dem Wasserdampf entnommen, wodurch dieser auskondensiert und als Kondensat über die vierte Verbindung dem Kreislauf zur Vorrichtung zur Dampferzeugung zurückgeführt wird. Damit steht der Vorrichtung zur Dampferzeugung Wasser aus dem Kondensat für eine erneute Aufnahme der thermischen Energie und Umwandlung in Wasserdampf zur Verfügung. Durch die Rückführung des Kondensats ist ein geringerer Wasserbedarf im Rahmen der Prozessführung durch Wiederverwendung und effektive Nutzung der Ausgangsstoffe gegeben.

Weiter bevorzugt weist die Vorrichtung zur Dampferzeugung eine Vorrichtung zur Abtrennung von Stickoxiden auf. Die bei der Verbrennung der Kohlenwasserstoffe und Sauerstoff, welcher beispielsweise in Form der Umgebungsluft zugeführt wird, entstehenden Stickoxide werden dabei als Abgas abgetrennt.

Die dritte Vorrichtung zur Generation von Wasserstoff, welche eine fünfte Verbindung zur zweiten Vorrichtung aufweist, ermöglicht es, den Wasserstoff in die zweite Vorrichtung einzubringen. Der dabei gebildete Wasserstoff und Sauerstoff wird in komprimierter Form (Druck > 15 bar) generiert, wobei jeweils eine mechanische Energie in Form von Druck an das Medium, Wasserstoff oder Sauerstoff, übertragen wird. Durch die Erzeugung von Wasserstoff und Sauerstoff in komprimierter Form kann die dabei gespeicherte innere Energie des Gases im System übertragen und in einer anderen Vorrichtung des Systems genutzt werden. Bevorzugt ist die Vorrichtung zur Generation von komprimiertem Wasserstoff modular ausgebildet und weist eine fünfte Verbindung zur zweiten Vorrichtung auf, die es ermöglicht, den Wasserstoff auf dem benötigten Druckniveau in die zweite Vorrichtung einzubringen.

Weiter bevorzugt ist die dritte Vorrichtung zur Generation von Wasserstoff als Elektrolysevorrichtung ausgebildet. Beispielsweise ist dadurch die Zuführung von Wasserstoff zur chemischen Synthese von Methanol möglich. Die Vorrichtung zur Generation von Wasserstoff weist einen Arbeitsdruck über dem der Methanolsynthese auf.

Dabei ist die Elektrolysevorrichtung bevorzugt zur elektrolytischen Erzeugung von Wasserstoff und Sauerstoff aus Wasser ausgebildet. Das hierfür eingesetzte Wasser kann dabei beispielsweise als Kondensat aus der ersten Vorrichtung oder als Produkt aus der zweiten Vorrichtung stammen. Durch die Einspeisung des in den anderen Prozessen gebildeten Wassers in die Elektrolysevorrichtung und dessen Nutzung zur Herstellung von Wasserstoff ist wiederum eine optimale Nutzung der eingesetzten Ausgangsstoffe innerhalb des Systems möglich.

Weiter bevorzugt ist die Elektrolysevorrichtung als alkalische Elektrolysevorrichtung oder PEM-Elektrolysevorrichtung ausgebildet. Die alkalische Elektrolyse nutzt modulare Stapel von Elektrolysezellen. Die Elektroden sind bevorzugt aus perforierten Stahlblechen mit einer möglichst porösen Oberfläche ausgebildet. Die Elektroden sind bevorzugt als Vorbleche nahe an dem Diaphragma positioniert und bevorzugt elektrisch leitend mit den Endplatten (Einzelzelle) bzw. den bipolaren Trennblechen (Zellstapel) verbunden. Zellrahmen dichten die Halbzellen bevorzugt nach außen ab und dienen als Einbettung für das Diaphragma. Die Stromquelle wird über die Endplatten kontaktiert. Beide Halbzellen sind bevorzugt mit einem alkalischen Elektrolyten geflutet bzw. werden von dieser Lauge durchströmt. Bevorratet wird die Lauge bevorzugt in separaten Tanks, die gleichzeitig als Gas-Flüssig-Separator dienen. Die PEM-Elektrolysezelle hingegen umfasst eine Anode (Sauerstoffproduktion) und eine Kathode (Wasserstoff-Produktion), welche durch eine saure Protonaustauschmembran (PEM, engl.: proton exchange membrane) voneinander getrennt sind.

Weiter bevorzugt weist das erfindungsgemäße System eine sechste Verbindung auf, die es ermöglicht, den in der Elektrolysevorrichtung gebildeten komprimierten Sauerstoff gegen den Kohlendioxidstrom zu entspannen und mit einem Druck <0,5 bar in die Vorrichtung zur Dampferzeugung einzuleiten. Dadurch ist eine Reduktion des Anteils an durch Umgebungsluft eingebrachten Sauerstoff möglich, was zudem zu einer Minimierung der Bildung von Stickoxiden führt. Alternativ wird der Wasserstoff mittels biologischer Verfahren, z. B. durch Algen oder Cyanobakterien, hergestellt. In diesem Fall ist die Vorrichtung zur Generation von Wasserstoff bevorzugt ein Photobioreaktor, welcher Algen oder Cyanobakterien, enthält.

In einer weiteren Ausführungsform der Erfindung umfasst das erfindungsgemäße System eine siebente Verbindung von der ersten Vorrichtung zur vierten Vorrichtung, die es ermöglicht, die in der exothermen biochemischen Reaktion entstehende Wärme der ersten Vorrichtung so an die vierte Vorrichtung abzugeben, das die benötigte Systemtemperatur in nichtproduktiven Phasen aufrecht erhalten wird. Dadurch kann die Menge an zugeführte Energie zur Aufrechterhaltung der Systemtemperatur vermindert bzw. vorteilhafterweise gänzlich verminderf werden. Beispielsweise erfolgt dadurch eine Nutzung der biologisch produzierten Wärme zur Aufrechterhaltung des Temperaturniveaus in der Elektrolyse

In einer weiteren Ausführungsform der Erfindung umfasst das erfindungsgemäßen System eine achte Verbindung von der vierten Vorrichtung zur zweiten Vorrichtung, die es ermöglicht, die für den chemisch katalytischen Prozess benötigte Systemtemperatur in der zweiten Vorrichtung temporär aufrecht zu erhalten. Durch die achte Verbindung ist es möglich, die in der vierten Vorrichtung vorgehaltene thermische Energie an die zweite Vorrichtung temporär zu übertragen, wodurch die in der zweiten Vorrichtung benötigte Systemtemperatur für den chemisch-katalytischen Prozess ohne Zuführung externer thermischer oder elektrischer Energie aufrecht erhalten werden kann. Dadurch wird eine weitere interne Nutzung der im System erzeugten thermischen Energie möglich.

In einer weiteren Ausführungsform der Erfindung stellen die im erfindungsgemäßen System hergestellten Paraffine, Alkene und Alkohole einen stofflichen Stromspeicher dar. Dabei können die erfindungsgemäß hergestellten Paraffine, Alkene und Alkoholebeispielsweise einer Verwertung, wie etwa einer Verbrennung zugeführt werden und über eine Turbine und einen Stromgenerator elektrische Energie erzeugt werden. Zudem können die erzeugten Paraffine, Alkene und Alkohole in flüssiger oder gasförmiger Form zur weiteren Verwendung dem System entzogen und einer Verwertung zur Stromerzeugung an einem anderen zugeführt werden.

In einer weiteren Ausführungsform der Erfindung erfolgt eine Verschaltung des abgestimmten Gesamtsystems mit einem Betriebsregime.

In einer weiteren Ausführungsform der Erfindung erfolgt ein Energietransfer von im Elektrolyseprozess erzeugten mechanischer Energie (Druck) im Volumenstrom des Sauerstoffs auf den Volumenstrom des Kohlendioxids.

In einer weiteren Ausführungsform der Erfindung erfolgt eine Stickstoffreduktion im Dampferzeuger durch die Sauerstoffnutzung.

Gegenstand der Erfindung ist auch ein Verfahren zur Herstellung von aliphatischen Alkoholen, umfassend die Schritte:
- Herstellung eines ersten aliphatischen Alkohols mittels eines biologischen Verfahrens, wobei Kohlenstoffdioxid gebildet wird,
- Umsetzung des gebildeten Kohlenstoffdioxids mittels einer chemischen Synthese, wobei
die Umsetzung des Kohlenstoffdioxids der Herstellung des ersten aliphatischen Alkohols nachgeschaltet ist und örtlich von dieser getrennt erfolgt. Unter örtlich getrennt wird dabei in unterschiedlichen Reaktionsbehältern verstanden, die jedoch durch Verbindungsleitungen miteinander verbunden sein können. Die Herstellung des ersten aliphatischen Alkohols und die Umsetzung des Kohlenstoffdioxids sind derart verbunden, dass das Kohlenstoffdioxid als Produkt der Herstellung des ersten aliphatischen Alkohols zumindest teilweise als Edukt zur Herstellung eines Kohlenwasserstoffs verwendet wird.

Im Sinne der vorliegenden Erfindung wird unter einem Kohlenwasserstoff eine Stoffgruppe oder ein Stoffgemisch von chemischen Verbindungen, die Kohlenstoff und Wasserstoff umfassen und Kohlenstoffketten, -ringe oder Kombinationen daraus bilden verstanden. Der Begriff Kohlenwasserstoff schließt ausdrücklich substituierte Verbindungen, insbesondere mit Substituenten ausgewählt aus sauerstoffhaltigen Gruppen (Hydroxy-, Ether-, und Estergruppen) ein. Die Kohlenwasserstoffe weisen bevorzugt 1 bis 20, besonders bevorzugt 1 bis 10, weiter bevorzugt 1 bis 5 C-Atome auf. Ganz besonders bevorzugt sind Kohlenwasserstoffe mit einem C-Atom. Unter einem kurzkettigen Kohlenwasserstoff werden solche mit 1 bis 6 C-Atomen verstanden.

Erfindungsgemäß ist der erste aliphatische Alkohol Ethanol. Bevorzugt erfolgt die Herstellung des ersten aliphatischen Alkohols durch Gärung mittels Mikroorganismen, wobei das in der Gärung gebildete Kohlendioxid als Edukt für die Herstellung des zweiten aliphatischen Alkohols verwendet wird. Dabei erfolgt die Herstellung des ersten aliphatischen Alkohols bevorzugt druckarm (<0,5bar).

Dabei erfolgt die Umsetzung des Kohlenstoffdioxids zu einem kurzkettigen Kohlenwasserstoff mittels an sich bekannten chemischen Synthesen in Gegenwart von Katalysatoren und Wasserstoff zu substituierten (bevorzugt sauerstoffhaltigen) oder unsubstituierten Kohlenwasserstoffen. Dabei wird bevorzugt als Zwischenschritt Synthesegas gebildet. Die Zusammensetzung des gebildeten kurzkettigen Kohlenwasserstoffs wird dabei in bekannter weise durch die Auswahl der Katalysatoren, der Wasserstoffkonzentration und die Reaktionsbedingungen gesteuert. Dabei erfolgt die chemischen Synthese unter hohem Druck (>=15bar) aus komprimiertem Wasserstoff und Kohlendioxid in einer exothermen Reaktion. Erfindungsgemäß erfolgt die Umsetzung des Kohlenstoffdioxids mittels chemischer Synthese mittels Fischer-Tropsch-Synthese. Die Fischer-Tropsch-Synthese ist eine exotherme Aufbaureaktion von CO/H₂-Gemischen an heterogenen-Katalysatoren zu Paraffinen, Alkenen und Alkoholen. Die Reaktion findet nur unter hohem Druck (20 - 40 bar) und bei einer Temperatur von 200°C - 350°C statt und verläuft nach folgenden allgemeinen Formeln:

nCO + (2n+1)H2 → CnH₂n+2+nH₂O (Alkane)

nCO + (2n)H₂ → CnH₂n+nH₂O (Alkene)

nCO+(2n)H₂ → CnH₂n+1OH⁺(n-1)H₂O (Alkohole)

Das Verfahren ist für die großtechnische Produktion von Benzin und Ölen von Bedeutung. Das typische Fischer-Tropsch -Produkt nach der Schulz-Flory-Verteilung enthält ca. 10-15% Flüssiggas, 50% Ottokraftstoff, 28% Dieselöle, 6% Weichparaffine und 2% Hartparaffine. Erfindungsgemäß umfasst das Verfahren weiterhin ein Verfahren zur Dampferzeugung, welches derart mit der Herstellung des ersten aliphatischen Alkohols verbunden ist, dass der im Verfahren zur Dampferzeugung generierte Dampf zum Eintrag thermischer Energie für die Herstellung des ersten aliphatischen Alkohols verwendet wird. Der bei der Dampferzeugung generierte Dampf, wird dabei beispielsweise aus Wasser gebildet, welches über die Verbrennung kohlenstoffhaltiger Verbindungen mit Sauerstoff verdampft wird. Die in Form des Wasserdampfs gespeicherte thermische Energie wird nunmehr bevorzugt zum Anmaischen, zur Temperierung bei der Herstellung des ersten aliphatischen Alkohols und/oder für die Abtrennung des ersten und/oder zweiten aliphatischen Alkohols eingesetzt. Durch die Generation von Dampf steht ein als Übertragungsmedium für thermische und mechanische Energie zur Verfügung welches für weitere Prozesse im System genutzt werden kann.

Weiter bevorzugt umfasst das Verfahren zur Dampferzeugung weiterhin die Abtrennung von Stickoxiden. Bei der Verbrennung von Sauerstoff, welcher beispielsweise in Form von Umgebungsluft zur Verbrennung eingesetzt wird, entstehen dabei Stickoxidverbindungen. Diese können zur Vermeidung des Einbringens in das System abgetrennt und als Abluft ausgetragen werden. Erfindungsgemäß umfasst das Verfahren weiterhin ein Verfahren zur Generation von Wasserstoff, welches derart mit der Herstellung von Paraffinen, Alkenen und Alkoholen verbunden ist, dass der generierte Wasserstoff als Edukt für die chemische Synthese der Paraffine, Alkene und Alkohole dient. Bevorzugt wird dabei aus Wasser Wasserstoff und Sauerstoff generiert, der Wasserstoff und Sauerstoff in komprimierter Form mit einem Druck > 15 bar erzeugt werden. Erfindungsgemäß ist das Verfahren zur Generation von Wasserstoff ein auf Elektrolyse basierendes Verfahren ausgewählt aus alkalischer Elektrolyse und PEM-Elektrolyse. Die alkalische Elektrolyse nutzt bevorzugt modulare Stapel von Elektrolysezellen. Die Elektroden sind bevorzugt aus perforierten Stahlblechen mit einer möglichst porösen Oberfläche ausgebildet. Die Elektroden sind bevorzugt als Vorbleche nahe an dem Diaphragma positioniert und elektrisch leitend mit den Endplatten (Einzelzelle) bzw. den bipolaren Trennblechen (Zellstapel) verbunden. Zellrahmen dichten bevorzugt die Halbzellen nach außen ab und dienen als Einbettung für das Diaphragma. Die Stromquelle wird bevorzugt über die Endplatten kontaktiert. Beide Halbzellen sind bevorzugt mit einem alkalischen Elektrolyten geflutet bzw. werden von dieser Lauge durchströmt. Bevorratet wird die Lauge in separaten Tanks, die gleichzeitig als Gas-Flüssig-Separator dienen.

Die PEM-Elektrolysezelle hingegen umfasst eine Anode (Sauerstoffproduktion) und einer Kathode (Wasserstoff-Produktion), welche durch eine saure Protonaustauschmembran (PEM, engl.: proton exchange membrane) voneinander getrennt sind. Erfindungsgemäß ist das Verfahren zur Generation von Wasserstoff ein auf der Elektrolyse von Wasser basierendes Verfahren. Dabei wird neben Wasserstoff durch die Elektrolyse auch Sauerstoff gebildet. Dieser Sauerstoff wird dabei für das Verfahren zur Dampferzeugung genutzt, wodurch der Anteil an Sauerstoff, welcher in Form von Umgebungsluft zur Verbrennung genutzt wird, minimiert oder vollständig ersetzt werden. Dieser Sauerstoff kann je nach Auslegung Luft als Oxidationsmittel ganz oder teilweise ersetzen. Dadurch verringert sich der Inertgasanteil. Der Energiebedarf zum Aufheizen dieser Bestandteile bis auf Zündtemperatur des Brennstoffes entfällt. Der Brennstoffbedarf sinkt. Die Effizienz der Dampferzeugung wird gesteigert. Ein weiterer Effekt zeigt sich durch die Begrenzung des Stickstoffeintrages. Der eingetragene Stickstoffanteil für jedes aus der Elektrolyse eingebrachte mol Sauerstoff sinkt von 3,76 mol bis maximal Null. Im Verbrennungsabgas sinkt der Stickoxidanteil deutlich.

Weiter bevorzugt wird das bei der Herstellung der Paraffine, Alkene und Alkohole gebildete Wasser für das Verfahren zur Generation von Wasserstoff eingesetzt. Dadurch kann der Bedarf an Wasser für die Elektrolyse verringert werden.

Weiter bevorzugt werden alle im Verfahren anfallenden Abwasserströme über die Herstellung des ersten aliphatischen Alkohols gesammelt. Dadurch können die Abwasserströme ökonomischer verbracht werden.

Das erfindungsgemäße Verfahren zeichnet sich durch die Kopplung eine ersten biologischen Verfahrens für die Herstellung von Ethanol und eines zweiten chemischsynthetischen Verfahren für die Herstellung von Paraffinen, Alkenen und Alkoholen aus, wobei CO₂ als Produkt des ersten Herstellungsverfahrens als Edukt für das zweite Herstellungsverfahren zumindest teilweise verwendet wird. Erfindungsgemäß umfasst das Verfahren daneben noch ein Verfahren zur Dampferzeugung, wobei der generierte Dampf als Übertragungsmedium für thermische und mechanische Energie für das Verfahren dient. Zudem umfasst das Verfahren auch ein Verfahren zur Generation von Wasserstoff, welcher als Edukt zur chemischen Synthese der Paraffine, Alkene und Alkohole dient. Zudem umfasst das Verfahren auch die Nutzung der inneren Energie des bei der Wasserstoffsynthese gebildeten komprimierten Wasserstoffs. Auch die über den Wasserdampf eingetragene thermische Energie ist ein weiteres Kennzeichen der systeminternen Nutzung der erzeugten Produkte und Energien.

Für die Realisierung der Erfindung ist es auch zweckmäßig die vorgenannten bevorzugten Ausgestaltungen und Ausführungsformen miteinander zu kombinieren. Bevorzugte Weiterbildungen der Erfindung ergeben sich aus den Kombinationen der Ansprüche oder einzelner Merkmale davon.

Nachfolgend sollen Aspekte der Erfindung anhand einiger Referenzbeispiele, die nicht in den Schutzbereich der Ansprüche fallen, und zugehöriger Figuren eingehender erläutert werden.

Es zeigen die
**Fig. 1** eine schematische Darstellung eines Systems gemäß einer ersten Ausgestaltung,
**Fig. 2** eine schematische Darstellung eines Systems gemäß einer zweiten Ausgestaltung und
**Fig. 3** eine schematische Darstellung eines Systems gemäß einer dritten Ausgestaltung.

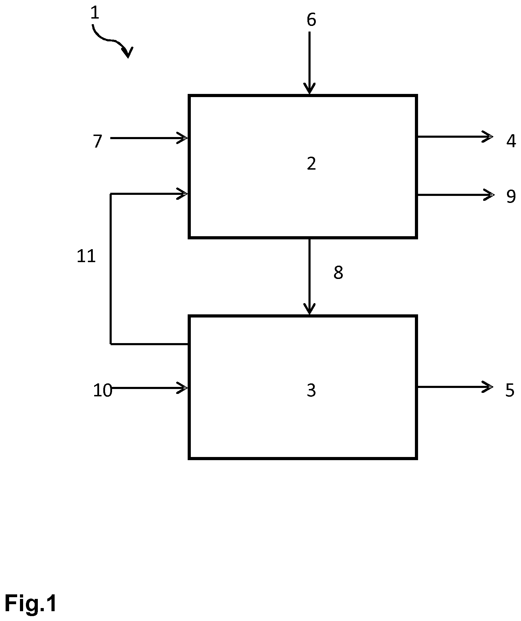

In der Fig. 1 ist beispielhaft ein System **1** gemäß einer ersten Ausgestaltung dargestellt. Das System **1** umfasst eine Vorrichtung **2** zur biologischen Herstellung eines ersten aliphatischen Alkohols **4** sowie eine Vorrichtung **3** zur chemischen Synthese eines Kohlenwasserstoffs 5, wie etwa eines zweiten aliphatischen Alkohols. Der erste aliphatische Alkohol **4** kann dabei Ethanol und der zweite aliphatische Alkohol **5** beispielsweise Methanol sein. Dabei kann das Ethanol **4** mittels alkoholischer Gärung durch Mikroorganismen gebildet werden. Hierzu werden Kohlenhydrate **6,** wie etwa Glucose, unter geeigneten Bedingungen und Wasser **7** in die Vorrichtung **2** eingebracht. Aufgrund der alkoholischen Gärung bildet sich neben Ethanol **4** auch Kohlendioxid **8.** Dieses Kohlendioxid **8** wird erfindungsgemäß für die chemische Synthese des Methanols **5** in der zweiten Vorrichtung **3** eingesetzt. Für die Synthese des Methanols **5** wird neben Kohlendioxid **8** auch Wasserstoff in die Vorrichtung **3** eingebracht und katalytisch zu Methanol **5** und Wasser **11** umgesetzt. Das gebildete Methanol **5** kann danach mittels einer nicht näher dargestellten Vorrichtung abgetrennt werden. Dies kann beispielsweise durch Destillation erfolgen. Das Wasser **11** kann nachfolgend für die alkoholische Gärung in die erste Vorrichtung **2** eingebracht und somit genutzt werden. Daneben fungiert das Wasser **11** auch als Energieträger, da es die bei der exothermen Reaktion der Methanolsynthese freigesetzte thermische Energie aufgenommen hat. Diese thermische Energie kann nun wiederum für die alkoholische Gärung in der ersten Vorrichtung **2** genutzt werden.

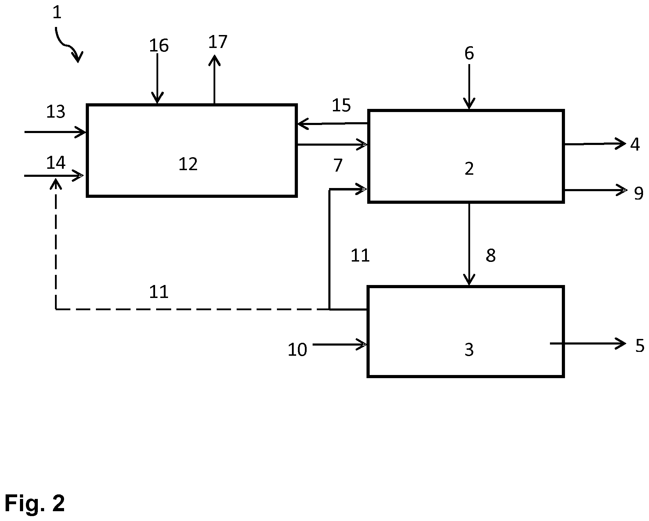

In der Fig. 2 ist beispielhaft ein System **1** gemäß einer zweiten Ausgestaltung dargestellt. Das System **1** entspricht dem vorher beschriebenen, wobei das System **1** nunmehr eine Vorrichtung zur Dampferzeugung **12** aufweist. In der Vorrichtung zur Dampferzeugung **12** werden kohlenstoffhaltige Verbindungen **16** unter Sauerstoff-Zufuhr **13** verbrannt. Der Sauerstoff **13** kann dabei beispielswiese als Umgebungsluft der Vorrichtung zur Verdampfung **12** zugeführt werden. Bei der Verbrennung entstehen Stickoxide **17,** welche abgetrennt und separat abgegeben werden. Die dabei entstehende thermische Energie wird auf das, der Vorrichtung **12** zugeführte Wasser **14** übertragen, wodurch diese verdampft und als Wasserdampf **7** der ersten Vorrichtung **2** zugeführt wird. Die im Wasserdampf enthaltene thermische Energie wird in der Vorrichtung **2** beispielsweise zur Temperierung oder zum Anmaischen verwendet, wobei dem Wasserdampf **7** beispielsweise über Wärmetauscher die thermische Energie entzogen wird. Infolgedessen kommt es zur Kondensation und das so gebildete Kondensat **15** kann dem Dampferzeuger zur erneuten Verdampfung zugeführt werden. Gleichfalls ist es auch denkbar, das in der zweiten Vorrichtung **3** bei der Methanolsynthese gebildete Wasser **11** der Vorrichtung zur Dampferzeugung **12** zuzuführen.

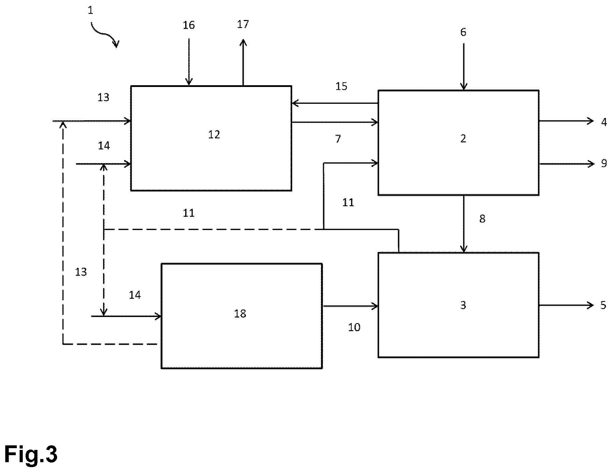

In der Fig. 3 ist beispielhaft ein System **1** gemäß einer dritten Ausgestaltung dargestellt. Das System **1** entspricht dem vorher beschriebenen, wobei das System zudem eine Vorrichtung zur Generation von Wasserstoff **10** umfasst. Dadurch muss der Wasserstoff 10 der zweiten Vorrichtung nicht mehr separat zugeführt werden, sondern kann im System **1** generiert werden. Der Wasserstoff **10** wird dabei beispielsweise mittels Elektrolyse von Wasser 14 gebildet, wodurch neben Wasserstoff **10** auch Sauerstoff **13** gebildet wird. Dieser Sauerstoff **13** kann für die Verbrennung der kohlenstoffhaltigen Verbindungen **16** in der Vorrichtung zur Dampferzeugung **12** genutzt werden. Dadurch verringert sich der Inertgasanteil. Der Energiebedarf zum Aufheizen dieser Bestandteile bis auf Zündtemperatur des Brennstoffes entfällt, weshalb der Brennstoffbedarf sinkt. Die Effizienz der Dampferzeugung wird dadurch gesteigert. Ein weiterer Effekt zeigt sich durch die Begrenzung des Stickstoffeintrages. Der eingetragene Stickstoffanteil für jedes aus der Elektrolyse eingebrachte mol Sauerstoff sinkt von 3,76 mol bis maximal Null. Im Verbrennungsabgas sinkt der Stickoxideanteil deutlich. Zudem kann das bei der Methanolsynthese gebildete Wasser **11** für die Elektrolyse in der Vorrichtung zur Wasserstoffgeneration **16** eingesetzt werden.

In einem weiteren nicht näher dargestellten Referenzbeispiel ist die Vorrichtung **3** zur chemischen Synthese eines Kohlenwasserstoffs **5** zur Bildung von Methan ausgebildet. Bei den Methansynthesen wird im Sabatier-Prozess katalytisch aus Kohlenstoffdioxid **8** und Wasserstoff **10,** Methan **5** und Wasser **11** gewonnen. Die Reaktionsformel lautet: CO₂ + 4 H₂ → CH₄ + 2 H₂O.

In einem weiteren Referenzbeispiel sind die theoretischen Stoffströme des Systems 1 bei der Bildung von Ethanol und Methanol berechnet worden.

Die folgende stöchiometrische Berechnung stellt ein theoretisches Verhältnis der Stoffströme zueinander auf Basis Einsatzgröße von 1.000kg Kohlendioxid dar.

**Beispiel Dampferzeugung**

| Eingang | | Ausgang | |
|---|---|---|---|
| Sauerstoff [g] | 1.090 kg | Kohlendioxid [g] | 1.499 kg |
| Kohlenstoff [s] | 409 kg | | |
| | | | |
| | 1.499 kg | | 1.499 kg |

**Beispiel Elektrolyse**

| Eingang | | Ausgang | |
|---|---|---|---|
| Wasser [I] | 1.227 kg | Wasserstoff [g] | 137 kg |
| | | Sauerstoff [g] | 1.090 kg |
| | | | |
| | 1.227 kg | | 1.227 kg |

**Beispiel Ethanolherstellung**

| Eingang | | Ausgang | |
|---|---|---|---|
| Glucose [s] | 2.046 kg | Kohlendioxid [g] | 1.000 kg |
| | | Ethylalkohol [I] | 1.046 kg |
| | | | |
| | 2.046 kg | | 2.046 kg |

**Beispiel Methanolsynthese**

| Eingang | | Ausgang | |
|---|---|---|---|
| Kohlendioxid [g] | 1.000 kg | Wasser [g] | 409 kg |
| Wasserstoff [g] | 137 kg | Methylalkohol [l] | 728 kg |
| | | | |
| | 1.137 kg | | 1.137 kg |

In einem weiteren Referenzbeispiel sind die berechneten Stoffströme im System für die Bildung von Ethanol und Methanol in der Tab.1 dargestellt. Die Abkürzung s steht für fest (solid), f für gasförmig und I für flüssig (liquid).

**Tab.1**

| Nr. | | Summenformel | Mittlere Molare Masse | Aggregatzustand | H₂ | N₂ | O₂ | Ar | CO₂ | C₆H₅OH | CH₄O | C₂H₆O | H₂O | sonst. |
|---|---|---|---|---|---|---|---|---|---|---|---|---|---|---|
| | | | [g/mol] | | [mol %] | [mol %] | [mol %] | [mol %] | [mol %] | [mol %] | [mol %] | [mol %] | [mol %] | [mol %] |
| 01 | Kohlenstoff, rein | C | 12,01 | s | --- | --- | --- | --- | --- | --- | --- | --- | --- | 100 |
| 02 | Luft, trocken | N₂, O₂, CO₂,... | 28,95 | g | | 78,08 | 20,94 | 0,93 | 0,03 | --- | --- | --- | --- | 0,002 |
| 03 | Wasser, vollentsalzt | H₂O | 18,02 | l | --- | --- | --- | --- | --- | --- | --- | --- | 100 | --- |
| 04 | Kohlendioxid | CO₂ | 44,01 | 9 | --- | --- | --- | --- | 100 | --- | --- | --- | --- | --- |
| 05 | Wasser | H₂O | 18,02 | l | --- | --- | --- | --- | --- | --- | --- | --- | 100 | --- |
| 06 | Wasser | H₂O | 18,02 | g | --- | --- | --- | --- | --- | --- | --- | --- | 100 | --- |
| 07 | Sauerstoff | O₂ | 15,99 | g | --- | --- | 100 | --- | --- | --- | --- | --- | --- | --- |
| 08 | Wasser | H₂O | 18,02 | l | --- | --- | --- | --- | --- | --- | --- | --- | 100 | --- |
| 09 | Wasserstoff | H₂ | 1,01 | g | 100 | --- | --- | --- | --- | --- | --- | --- | --- | --- |
| 10 | Glucose | C₆H₁₂O₆ | 180,15 | s | --- | --- | --- | --- | --- | 100 | --- | --- | --- | --- |
| 11 | Kohlendioxid | CO₂ | 44,01 | | --- | --- | --- | --- | 100 | --- | --- | --- | --- | --- |
| 12 | Ethylalkohol | C2H6O | 46,07 | l | --- | --- | --- | --- | --- | --- | --- | 99,95 | --- | 0,05 |
| 13 | Abwasser | H2O | 18,02 | l | --- | --- | --- | --- | --- | --- | --- | --- | 70 | 30 |
| 14 | Wasser | H2O | 18,02 | g | --- | --- | --- | --- | --- | --- | --- | --- | 100 | --- |
| 15 | Methylalkohol | CH4O | 32,04 | l | --- | --- | --- | --- | --- | --- | 99,95 | --- | --- | 0,05 |
| 16 | Luft- SauerstoffGemisch | N2, O2, CO2,... | 28.95 | g | | < 78,08 | > 20,94 | < 0,93 | < 0,03 | --- | --- | --- | --- | < 0,002 |
| 17 | Wasser, vollentsalzt | H2O | 18,02 | l | --- | --- | --- | --- | --- | --- | --- | --- | 100 | --- |

In einem weiteren Referenzbeispiel sind die theoretischen Stoffströme des Systems 1 bei der Bildung von Ethanol und Methan berechnet worden.

Die theoretische Umsetzung der Massenverhältnisse ergibt sich wie folgt:

| **Eingang** | | **Ausgang** | |
|---|---|---|---|
| Kohlendioxid [g] | 845 kg | Wasser [g] | 692 kg |
| Wasserstoff [g] | 155 kg | Methan [g] | 308 kg |
| | 1.000 kg | | 1.000 kg |

Die Reaktion der Stoffumwandlung ist stark exotherm. Die thermische Energie wird über die Umwandlung des entstehenden Wassers zu Wasserdampf abgeleitet. Energetisch betrachtet, wird die chemische Energie aus dem Wasserstoff und dem Kohlendioxid in chemische Energie des Methans umgesetzt. Dabei entstehen Verluste durch Abgabe von thermischer Energie. Der eingesetzte Katalysator ist in der Regel nicht besonders aktiv. Verbesserte Katalysatoreigenschaften werden durch Zuführung von mechanischer und thermischer Energie erzielt.

In einem weiteren Referenzbeispiel sind nachfolgend stöchiometrische Berechnungen für ein theoretisches Verhältnis der Stoffströme bei Ethanol und Methanbildung zueinander auf Basis Einsatzgröße von 1.000kg Kohlendioxid dargestellt.

**Dampferzeugung**

| **Eingang** | | **Ausgang** | |
|---|---|---|---|
| Sauerstoff [g] | 1.090 464 kg | Kohlendioxid [g] | 1.4992.013 kg |
| Kohlenstoff [s] | 409 549 kg | | |
| | 1.4992.013 kg | | 2.0131.499 kg |

**Elektrolyse**

| **Eingang** | | **Ausgang** | |
|---|---|---|---|
| Wasser [I] | 1.227 648 kg | Wasserstoff [g] | 137 184 kg |
| | | Sauerstoff [g] | 1.090 464 kg |
| | 1.227 648 kg | | 1.227 648 kg |

**Ethanolherstellung**

| **Eingang** | | **Ausgang** | |
|---|---|---|---|
| Glucose [s] | 2.046 kg | Kohlendioxid [g] | 1.000 kg |
| | | Ethylalkohol [I] | 1.046 kg |
| | 2.046 kg | | 2.046 kg |

**Methansynthese**

| **Eingang** | | **Ausgang** | |
|---|---|---|---|
| Kohlendioxid [g] | 1.000 kg | Wasser [g] | 409 819 kg |
| Wasserstoff [g] | 137 184 kg | Methan [g] | 728 365 kg |
| | 1.137 184 kg | | 1.137 184 kg |

In einem weiteren Referenzbeispiel sind die berechneten Stoffströme im System für die Bildung von Ethanol und Methan in der Tab.2 dargestellt.

**Tab. 2**

| Nr | Stoff | Summenformel | Mittlere Molare Masse | Aggregatzustand | H₂ | N₂ | O₂ | Ar | CO₂ | C₆H₅OH | CH₄ | C₂H₆O | H₂O | sonst. |
|---|---|---|---|---|---|---|---|---|---|---|---|---|---|---|
| | | | [g/mol] | | [mol %] | [mol %] | [mol %] | [mol %] | [mol %] | [mol %] | [mol %] | [mol %] | [mol %] | [mol %] |
| 01 | Kohlenstoff, rein | C | 12,01 | s | --- | --- | --- | --- | --- | --- | --- | --- | --- | 100 |
| 02 | Luft, trocken | N₂, O₂, CO₂,... | 28,95 | g | --- | 78,08 | 20,94 | 0,93 | 0,03 | --- | --- | --- | --- | 0,002 |
| 03 | Wasser, vollentsalzt | H₂O | 18,02 | l | --- | --- | --- | --- | --- | --- | --- | --- | 100 | --- |
| 04 | Kohlendioxid | CO₂ | 44,01 | g | --- | --- | --- | --- | 100 | --- | --- | --- | --- | --- |
| 05 | Wasser | H₂O | 18,02 | l | --- | --- | --- | --- | --- | --- | --- | --- | 100 | --- |
| 06 | Wasser | H₂O | 18,02 | g | --- | --- | --- | --- | --- | --- | --- | --- | 100 | --- |
| 07 | Sauerstoff | O₂ | 15,99 | g | --- | --- | 100 | --- | --- | --- | --- | --- | --- | --- |
| 08 | Wasser | H₂O | 18,02 | l | --- | --- | --- | --- | --- | --- | --- | --- | 100 | --- |
| 09 | Wasserstoff | H₂ | 1,01 | g | 100 | --- | --- | --- | --- | --- | --- | --- | --- | --- |
| 10 | Glucose | C₆H₁₂O₆ | 180,15 | s | --- | --- | --- | --- | --- | 100 | --- | --- | --- | --- |
| 11 | Kohlendioxid | CO₂ | 44,01 | g | --- | --- | --- | --- | 100 | --- | --- | --- | --- | --- |
| 12 | Ethylalkohol | C₂H₆O | 46,07 | l | --- | --- | --- | --- | --- | --- | --- | 99,95 | --- | 0,05 |
| 13 | Abwasser | H₂O | 18,02 | l | --- | --- | --- | --- | --- | --- | --- | --- | 70 | 30 |
| 14 | Wasser | H₂O | 18,02 | g | --- | --- | --- | --- | --- | --- | --- | --- | 100 | --- |
| 15 | Methan | CH₄ | 16,04 | g | --- | --- | --- | --- | --- | --- | 99,95 | --- | --- | 0,05 |
| 16 | Luft- Sauerstoff-Gemisch | N₂, O₂, CO₂,... | 28,95 | g | --- | < 78,08 | > 20,94 | < 0,93 | < 0,03 | --- | --- | --- | --- | < 0,002 |
| 17 | Wasser, vollentsalzt | H₂O | 18,02 | l | --- | --- | --- | --- | --- | --- | --- | --- | 100 | --- |

### Bezugszeichenliste

- 1: System
- 2: Vorrichtung zur biologischen Herstellung eines ersten aliphatischen Alkohols, bevorzugt Ethanol
- 3: Vorrichtung zur chemischen Synthese eines Kohlenwasserstoffes,
- 4: erster aliphatischer Alkohol, bevorzugt Ethanol
- 5: Kohlenwasserstoff, bevorzugt Methanol oder Methan
- 6: Kohlenhydrate
- 7: Wasser
- 8: Kohlendioxid
- 9: Prozessabwässer
- 10: Wasserstoff
- 11: Wasser als Produkt der chemischen Synthese des Kohlenwasserstoffs
- 12: Vorrichtung zur Dampferzeugung
- 13: Sauerstoff
- 14: Wasser
- 15: Kondensat
- 16: Kohlenstoffhaltige Verbindungen
- 17: Stickoxidhaltige Abgase
- 18: Vorrichtung zur Generation von Wasserstoff

## Patentansprüche

1. System (1) zur Erzeugung von aliphatischen Alkoholen umfassend:
- zumindest eine erste Vorrichtung (2) zur biologischen Herstellung von Ethanol (4), wobei Kohlenstoffdioxid gebildet wird und
- zumindest eine zweite Vorrichtung (3) zur Umsetzung des Kohlenstoffdioxids (8) mittels chemischer Synthese mittels Fischer-Tropsch-Synthese, welche eine exotherme Aufbaureaktion von CO/H₂-Gemischen an heterogenen Katalysatoren zu Paraffinen, Alkenen und Alkoholen ist,
wobei die zweite Vorrichtung (3) zur chemischen Synthese mittels Fischer-Tropsch-Synthese der ersten Vorrichtung (2) zur biologischen Herstellung des Ethanols (4) nachgeschaltet ist, sowie eine erste Verbindung von der ersten Vorrichtung (2) zur zweiten Vorrichtung (3), die es ermöglicht, ein in der ersten Vorrichtung (2) gebildetes Kohlenstoffdioxid (8) in die zweite Vorrichtung (3) einzuleiten, wobei
- das System eine zweite Verbindung von der zweiten Vorrichtung (3) zur ersten Vorrichtung (2) aufweist, die es ermöglicht, in der zweiten Vorrichtung (3) gebildetes Wasser (11) in die erste Vorrichtung (2) einzuleiten und zum Anmaischen zu verwenden,
- zumindest eine Vorrichtung zur Dampferzeugung (12) und eine dritte Verbindung, wobei die dritte Verbindung zwischen der Vorrichtung zur Dampferzeugung (12) und der ersten Vorrichtung (2) angeordnet ist und es ermöglicht, den in der Vorrichtung zur Dampferzeugung (12) gebildeten Dampf (7) in die erste Vorrichtung (2) einzuleiten,
- zumindest eine dritte Vorrichtung zur Generation von Wasserstoff (16), welche eine fünfte Verbindung zur zweiten Vorrichtung (3) aufweist, die es ermöglicht, den Wasserstoff (10) in die zweite Vorrichtung (3) einzubringen, wobei die dritte Vorrichtung (16) eingerichtet ist, aus Wasser (14) Wasserstoff (10) und Sauerstoff (13) zu generieren, wobei die Vorrichtung eingerichtet ist, komprimierten Wasserstoff und Sauerstoff mit einem Druck > 15 bar zu generieren,
- das System (1) eine vierte Verbindung von der ersten Vorrichtung (2) zur Vorrichtung zur Dampferzeugung (12) aufweist, die es ermöglicht, ein in der ersten Vorrichtung (2) gebildetes Kondensat (15) in die Vorrichtung zur Dampferzeugung (12) einzuleiten,
- die dritte Vorrichtung der zweiten Vorrichtung (3) komprimierten Wasserstoff mit einem Druck > 15 bar zur Verfügung stellt,
- das System (1) eine vierte Vorrichtung umfasst, die zur Vorhaltung thermischer Energie ausgebildet ist, welche derart mit der Herstellung des Ethanols (4) verbunden ist, dass der generierte Dampf (7) zum Eintrag thermischer Energie für die Herstellung des Ethanols (4) in der ersten Vorrichtung (2) verwendet wird.

2. System nach Anspruch 1, **dadurch gekennzeichnet, dass** die Vorrichtung zur Generation von Wasserstoff (16) als Elektrolysevorrichtung ausgebildet ist.

3. System nach Anspruch 2, **dadurch gekennzeichnet, dass** das System (1) eine sechste Verbindung aufweist, die es ermöglicht, den in der Elektrolysevorrichtung (16) gebildeten Sauerstoff (13) in die Vorrichtung zur Dampferzeugung (12) einzuleiten.

4. System nach einem der vorhergehenden Ansprüche, **dadurch gekennzeichnet, dass** die erste Vorrichtung (2) zur druckarmen Herstellung des Ethanols (4) bei < 0,5 bar ausgebildet ist.

5. System nach einem der vorhergehenden Ansprüche, umfassend eine siebente Verbindung von der ersten Vorrichtung (2) zur vierten Vorrichtung, die es ermöglicht, die in der exothermen biochemischen Reaktion entstehende Wärme der ersten Vorrichtung (2) so an die vierte Vorrichtung abzugeben, dass das Temperaturniveau in der Elektrolyse in nichtproduktiven Phasen aufrecht erhalten wird.

6. System nach einem der vorhergehenden Ansprüche, umfassend eine achte Verbindung von der vierten Vorrichtung zur zweiten Vorrichtung (3), die es ermöglicht, die für den chemisch katalytischen Prozess benötigte Systemtemperatur in der zweiten Vorrichtung (3) temporär aufrecht zu erhalten.

7. Verfahren zur Herstellung von aliphatischen Alkoholen, umfassend:
- Herstellung von Ethanol (4) mittels eines biologischen Verfahrens, wobei Kohlenstoffdioxid (8) gebildet wird,
- Umsetzung des Kohlenstoffdioxids (8) mittels chemischer Synthese mittels Fischer-Tropsch-Synthese, wobei die Fischer-Tropsch-Synthese eine exotherme Aufbaureaktion von CO/H₂-Gemischen an heterogenen-Katalysatoren zu Paraffinen, Alkenen und Alkoholen ist und die Reaktion unter hohem Druck von 20 - 40 bar und bei einer Temperatur von 200°C - 350°C stattfindet,
wobei die Umsetzung des Kohlenstoffdioxids (8) der Herstellung von Ethanol (4) nachgeschaltet und örtlich getrennt ist und die Herstellung von Ethanol (4) und die Umsetzung des Kohlenstoffdioxids (8) derart verbunden sind, dass das Kohlenstoffdioxid (8) als Produkt der Herstellung von Ethanol (4) zumindest teilweise als Edukt zur Herstellung von Paraffinen, Alkenen und Alkoholen verwendet wird sowie ein Verfahren zur Dampferzeugung, welches derart mit der Herstellung des Ethanols (4) verbunden ist, dass der im Verfahren zur Dampferzeugung generierte Dampf (7) zum Eintrag thermischer Energie für die Herstellung des Ethanols (4) verwendet wird und ein Verfahren zur Generation von Wasserstoff, welches derart mit der Herstellung von Paraffinen, Alkenen und Alkoholen verbunden ist, dass der generierte Wasserstoff (10) als Edukt für die chemische Synthese von Paraffinen, Alkenen und Alkoholen dient, wobei das Verfahren zur Generation von Wasserstoff ein auf der Elektrolyse von Wasser (14) basierendes Verfahren ist, bei dem neben Wasserstoff (10) durch die Elektrolyse auch Sauerstoff (13) gebildet wird, welcher für das Verfahren zur Dampferzeugung genutzt wird, wobei das Verfahren zur Generation von Wasserstoff ausgewählt ist aus alkalischer Elektrolyse und PEM-Elektrolyse.

8. Verfahren nach Anspruch 7, **dadurch gekennzeichnet, dass** ein Energietransfer von im Elektrolyseprozess erzeugter mechanischer Energie im Volumenstrom des Sauerstoffs auf den Volumenstrom des Kohlenstoffdioxids erfolgt.

## Claims

1. A system (1) for producing aliphatic alcohols, comprising:
- at least one first device (2) for the biological production of ethanol (4), wherein carbon dioxide is formed, and
- at least one second device (3) for chemically reacting the carbon dioxide (8) by means of chemical synthesis in a Fischer-Tropsch synthesis method, which is an exothermic "Aufbaureaktion" of CO/H₂ mixtures on heterogeneous catalysts for forming paraffins, alkenes, and alcohols,
wherein the second device (3) for chemical synthesis in a Fischer-Tropsch synthesis method is arranged downstream of the first device (2) for the biological production of ethanol (4), and a first connection from the first device (2) to the second device (3) via which it is possible to pass the carbon dioxide (8) formed in the first device (2) into the second device (3), wherein
- the system includes a second connection from the second device (3) to the first device (2) via which it water (11) formed in the second device (3) can be passed into the first device (2) and used to create a mash,
- at least one device (12) for producing steam and a third connection, wherein the third connection is disposed between the steam production device (12) and the first device (2) via which it is possible for the steam (7) formed in the steam production device (12) to be passed into the first device (2),
- at least one third device (16) for generating hydrogen, which includes a fifth connection to the second device (3), and via which it is possible to introduce the hydrogen (10) into the second device (3), wherein the third device (16) is designed to generate hydrogen (10) and oxygen (13) from water (14), wherein the device is designed to generate compressed hydrogen and oxygen with a pressure of > 15 bar,
- the system (1) includes a fourth connection from the first device (2) to the steam production device (12), via which it is possible to pass a condensate (15) formed in the first device (2) into the steam production device (12),
- the third device supplied the second device (3) with compressed hydrogen under pressure of > 15 bar
- the system (1) comprises a fourth device which is designed to hold a reserve of thermal energy, and which is connected to the production system for ethanol (4) in such manner that the the generated steam (7) is used to contribute thermal energy for production of the ethanol (4) in the first device (2).

2. The system according to claim 1, **characterised in that** the device (16) for generating hydrogen is designed as an electrolysis device.

3. The system according to claim 2, **characterised in that** the system (1) includes a sixth connection via which it is possible to introduce the oxygen (13) formed in the electrolysis device (16) into the steam production device (12).

4. The system according to any one of the preceding claims, **characterised in that** the first device (2) is designed for low pressure production of ethanol, at < 0.5 bar.

5. The system according to any one of the preceding claims, comprising a seventh connection from the first device (2) to the fourth device, via which it is possible to deliver heat generated in the exothermic biochemical reaction from the first device (2) to the fourth device in such manner that the temperature level is maintained in non-productive phased during the electrolysis.

6. The system according to any one of the preceding claims, comprising an eighth connection, from the fourth device to the second device (3), via which it is possible to maintain the system temperature required for the chemical catalytic process temporarily in the second device (3).

7. A method for producing aliphatic alcohols, comprising:
- producing ethanol (4) by means of a biological process, wherein carbon dioxide (8) is formed,
- reacting the carbon dioxide (8) by means of chemical synthesis in a Fischer-Tropsch synthesis method, wherein Fischer-Tropsch synthesis method is an exothermic "Aufbaureaktion" of CO/H₂ mixtures on heterogeneous catalysts for forming paraffins, alkenes, and alcohols and the reaction takes place under high pressure from 20 - 40 bar and at a temperature from 200 °C - 350 °C,
wherein the reaction of the carbon dioxide (8) is arranged downstream of the production of ethanol (4) and separately therefrom, and the production of ethanol (4) and the reaction of the carbon dioxide (8) are connected in such manner that at least some of the carbon dioxide (8) as the product of the production of ethanol (4) is used as a reactant for producing paraffins, alkenes, and alcohols, and a method for producing steam which is connected to the system for producing ethanol (4) in such manner that the steam (7) formed in the steam production method is used to contribute thermal energy for the production of ethanol (4), and a method for generating hydrogen which is connected to the system for producing paraffins, alkenes, and alcohols in such manner that the hydrogen (10) generated serves as a reactant for the chemical synthesis of paraffins, alkenes, and alcohols, wherein the method for generating hydrogen is a method based on the electrolysis of water (14), in which oxygen (13) is also formed by the electrolysis besides hydrogen (10), which oxygen is use for the steam production process, wherein the method for generating hydrogen is chosen from alkaline electrolysis and PEM electrolysis.

8. The method according to claim 7, **characterised in that** mechanical energy generated in the volume flow of the oxygen during the electrolysis process is transferred to the volume flow of the carbon dioxide.

## Revendications

1. Système (1) de production d'alcools aliphatiques, comprenant :
- au moins un premier dispositif (2) de fabrication biologique d'éthanol (4) impliquant la formation de dioxyde de carbone, et
- au moins un deuxième dispositif (3) permettant de faire réagir le dioxyde de carbone (8) en mettant en oeuvre une synthèse chimique par synthèse Fischer-Tropsch, s'agissant d'une réaction exothermique permettant d'obtenir des paraffines, des alcènes et des alcools en passant des mélanges de CO/H₂ sur des catalyseurs hétérogènes,
le deuxième dispositif (3) de synthèse chimique par synthèse Fischer-Tropsch étant disposé en aval du premier dispositif (2) de fabrication biologique de l'éthanol (4), ainsi qu'une première conduite de liaison allant du premier dispositif (2) vers le deuxième dispositif (3), permettant ainsi d'introduire dans le deuxième dispositif (3) du dioxyde de carbone (8) formé dans le premier dispositif (2),
- ledit système comportant une deuxième conduite de liaison allant du deuxième dispositif (3) vers le premier dispositif (2), laquelle permet d'introduire dans le premier dispositif (2) de l'eau (11) formée dans le deuxième dispositif (3) et de l'utiliser pour préparer le mélange à fermenter,
- au moins un dispositif de production de vapeur (12) et une troisième conduite de liaison, la troisième conduite de liaison étant disposée entre le dispositif de production de vapeur (12) et le premier dispositif (2), permettant ainsi d'introduire dans le premier dispositif (2) la vapeur (7) formée dans le dispositif de production de vapeur (12),
- au moins un troisième dispositif de production d'hydrogène (16), comportant une cinquième conduite de liaison vers le deuxième dispositif (3), laquelle permet d'introduire l'hydrogène (10) dans le deuxième dispositif (3), le troisième dispositif (16) étant réalisé de manière à produire de l'hydrogène (10) et de l'oxygène (13) à partir d'eau (14), ledit dispositif permettant de produire de l'hydrogène et de l'oxygène comprimés sous une pression > 15 bar,
- le système (1) comportant une quatrième conduite de liaison allant du premier dispositif (2) vers le dispositif de production de vapeur (12), laquelle permet d'introduire dans le dispositif de production de vapeur (12) un condensat (15) formé dans le premier dispositif (2),
- le troisième dispositif fournissant au deuxième dispositif (3) de l'hydrogène comprimé sous une pression > 15 bar,
- le système (1) comprenant un quatrième dispositif réalisé de manière à pouvoir stocker de l'énergie thermique tout en étant relié à la fabrication d'éthanol (4) pour ainsi permettre d'utiliser la vapeur (7), suite à sa génération, pour fournir de l'énergie thermique servant à produire l'éthanol (4) au sein du premier dispositif (2).

2. Système selon la revendication 1, **caractérisé en ce que** le dispositif de génération d'hydrogène (16) est réalisé sous forme d'un dispositif d'électrolyse.

3. Système selon la revendication 2, **caractérisé en ce que** le système (1) comporte une sixième conduite de liaison permettant d'introduite l'oxygène (13), suite à sa formation dans le dispositif d'électrolyse (16), dans le dispositif de production de vapeur (12).

4. Système selon l'une des revendications précédentes, **caractérisé en ce que** le premier dispositif (2) est réalisé de manière à produire l'éthanol (4) à une faible pression < 0,5 bar.

5. Système selon l'une des revendications précédentes, comprenant une septième conduite de liaison allant du premier dispositif (2) vers le quatrième dispositif, laquelle permet de transférer au quatrième dispositif la chaleur produite lors de la réaction biochimique exothermique dans le premier dispositif (2), pour ainsi maintenir la température dans l'électrolyse pendant les phases hors production.

6. Système selon l'une des revendications précédentes, comprenant une huitième conduite de liaison allant du quatrième dispositif vers le deuxième dispositif (3), laquelle permet de temporairement maintenir dans le deuxième dispositif (3) la température du système telle qu'elle est requise pour le processus chimique catalytique.

7. Procédé de fabrication d'alcools aliphatiques, comprenant :
- la fabrication d'éthanol (4) au moyen d'un procédé biologique impliquant la formation de dioxyde de carbone (8),
- la réaction du dioxyde de carbone (8) au moyen d'une synthèse chimique par synthèse Fischer-Tropsch, la synthèse Fischer-Tropsch étant une réaction exothermique permettant d'obtenir des paraffines, des alcènes et des alcools en passant des mélanges de CO/H₂ sur des catalyseurs hétérogènes, ladite réaction étant menée sous une haute pression comprise entre 20 et 40 bar et à une température comprise entre 200 °C et 350 °C,
la réaction du dioxyde de carbone (8) se déroulant en aval de la fabrication d'éthanol (4) et dans un endroit séparé de cette dernière, la fabrication d'éthanol (4) et la réaction du dioxyde de carbone (8) étant reliées de manière à utiliser le dioxyde de carbone (8), constituant un produit de la fabrication d'éthanol (4), au moins partiellement comme produit de départ pour de la fabrication de paraffines, d'alcènes et d'alcools, ainsi qu'un procédé de production de vapeur lequel est relié à la fabrication de l'éthanol (4) de manière à ce que la vapeur (7), générée dans le procédé de production de vapeur, est utilisée pour fournir de l'énergie thermique à la production de l'éthanol (4), et un procédé de génération d'hydrogène, lequel est relié à la fabrication de paraffines, d'alcènes et d'alcools de manière à ce que l'hydrogène (10) soit utilisé, suite à sa génération, comme produit de départ pour la synthèse chimique de paraffines, d'alcènes et d'alcools, le procédé de génération d'hydrogène étant un procédé basé sur l'électrolyse de l'eau (14) dans lequel ladite électrolyse engendre la formation non seulement de l'hydrogène (10) mais aussi de l'oxygène (13), ce dernier étant mis en oeuvre dans le procédé de production de vapeur, le procédé de production d'hydrogène étant choisi parmi l'électrolyse alcaline et l'électrolyse PEM.

8. Procédé selon la revendication 7, **caractérisé en ce que** de l'énergie mécanique, produite dans le processus d'électrolyse et contenue dans le débit volumique de l'oxygène, fait l'objet d'un transfert d'énergie sur le débit volumique du dioxyde de carbone.
